(19)

Europäisches Patentamt
European Patent Office
Office européen des brevets

(11)  **EP 4 721 681 A1**

(12)  **EUROPEAN PATENT APPLICATION**
published in accordance with Art. 153(4) EPC

(43) Date of publication:
08.04.2026  Bulletin 2026/15

(51) International Patent Classification (IPC):
**A61B 17/32** (2006.01)

(21) Application number: 24869906.8

(22) Date of filing: 27.06.2024

(86) International application number:
PCT/CN2024/101863

(87) International publication number:
WO 2025/066347 (03.04.2025 Gazette 2025/14)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH MA MD TN

(30) Priority: 26.09.2023  CN 202311243079

(71) Applicant: Innolcon Medical Technology (Suzhou)
Co., Ltd.
Jiangsu 215000 (CN)

(72) Inventors:
• WANG, Lei
Suzhou, Jiangsu 215000 (CN)
• YAN, Zhongyu
Suzhou, Jiangsu 215000 (CN)
• LUO, Wei
Suzhou, Jiangsu 215000 (CN)

(74) Representative: Ullrich & Naumann PartG mbB
Schneidmühlstrasse 21
69115 Heidelberg (DE)

(54)  **ULTRASONIC SCALPEL BLADE**

(57)  The present disclosure relates to the field of medical instruments. Provided is an ultrasonic scalpel blade applied to ultrasonic scalpel comprising a clamp. The blade comprises a connecting rod and a blade body, wherein the blade also has a cutting groove located on the connecting rod and extending towards the blade body. The blade body includes a tissue cutting side, a back cutting side, a first arc side, and a second arc side. The tissue cutting side and the back cutting side are opposite to each other, as are the first and second arc sides. The distal end of the blade body is offset from the central axis of the connecting rod. The back cutting side comprises a first cutting surface and a second cutting surface forming a cutting edge. Through structural design, the blade has a certain curvature, enabling it to be used in surgeries on human tissues with a certain curvature, reducing incision and thermal damage in operation. The cutting groove and other structures on the blade can be matched with the curvature parameters of the blade body, ensuring stable vibration modes when the large-curvature blade operates at the designed resonant frequency, thus improving surgical safety.

FIG 1

## Description

The present application claims the priority to Chinese Patent Application No. 202311243079.6 filed on September 26, 2023, and the entire contents of which are incorporated herein by reference.

## FIELD OF THE INVENTION

The present disclosure relates to the field of medical instruments, and in particular to an ultrasonic scalpel blade.

## BACKGROUND OF THE INVENTION

The ultrasonic scalpel is a surgical instrument that uses an ultrasonic transducer to generate ultrasonic vibration waves, which then drive the scalpel blade to cut and coagulate human tissue. Clinically, this instrument can remove lesions at lower temperatures and with less bleeding, while ensuring minimal lateral thermal damage to the tissue.

Because tissue shapes differ in different areas, the cutting effect and thermal damage caused by the same ultrasonic scalpel blade will vary. Therefore, the same blade cannot be used to treat all tissue sites. To reduce incision and thermal damage caused by the ultrasonic scalpel during surgery, blades with different shapes are required when cutting and coagulating tissues in different areas by the ultrasonic scalpel.

However, since ultrasonic scalpels are driven by ultrasonic vibration waves, blades with different shapes, especially blades with large curvature, will affect the overall resonant frequency and vibration mode of the ultrasonic scalpel, reducing its working stability. Furthermore, some tissues with large curvature, such as thyroid tissues, require blades with large curvature during surgery to reduce incision and thermal damage. Therefore, there is an urgent need for an ultrasonic scalpel that can improve the curvature and working length while maintaining working stability.

## SUMMARY OF THE INVENTION

The present disclosure provides an ultrasonic scalpel blade to solve the problems of unstable vibration modes and low operational stability of ultrasonic scalpel blades with high curvature and long working length.

A first aspect of the embodiment of the present disclosure provides an ultrasonic scalpel blade, applied to an ultrasonic scalpel including a clamp, wherein the blade includes a connecting rod and a blade body sequentially from proximal to distal end. The blade has a cutting groove located on the connecting rod and extending towards the blade body. The blade body includes a tissue cutting side, a back cutting side, a first arc side, and a second arc side. The tissue cutting side and the back cutting side are arranged opposite to each other, and the first arc side and the second arc side are arranged opposite to each other. The tissue cutting side is used to cut tissue and/or cooperate with the clamp to clamp tissue, thereby achieving tissue separation and hemostasis. The first and second arc sides are located on two sides of the tissue cutting side. The tissue cutting side has a curved surface structure, and the first arc side and second arc side are perpendicular to the plane formed by the two sides of the curved surface structure. The distal end of the blade body is offset from the central axis of the connecting rod. The back cutting side includes a first cutting surface and a second cutting surface, with the first cutting surface being located between the first arc side and the second cutting surface to form a cutting edge with the second cutting surface.

A second aspect of the embodiment of the present disclosure provides an ultrasonic scalpel, including a clamp, the ultrasonic scalpel blade as described above, a blade shaft, a transducer, and a sheath, wherein the blade includes a connecting rod and a blade body. The connecting rod is disposed at the distal end of the blade shaft, and the blade body is disposed at the distal end of the connecting rod. The proximal end of the blade shaft is connected to the transducer. The sheath is sleeved outside the blade shaft, the proximal end of the sheath is connected to the transducer, and the distal end of the sheath is connected to the clamp.

A third aspect of the embodiment of the present disclosure also provides an ultrasonic scalpel, including a clamp, the ultrasonic scalpel blade as described above, and a transducer, wherein the blade includes a connecting rod and a blade body disposed at the distal end of the connecting rod. The proximal end of the connecting rod is connected to the transducer, and the clamp is hinged to the transducer so that the clamp cooperate with the blade body to clamp tissue.

As can be seen from the above technical solution, the present application provides an ultrasonic scalpel blade, applied to an ultrasonic scalpel including a clamp. The blade includes a connecting rod and a blade body sequentially from the proximal end to the distal end. The blade has a cutting groove located on the connecting rod and extending towards the blade body. The blade body includes a tissue cutting side, a back cutting side, a first arc side, and a second arc side. The tissue cutting side and the back cutting side are arranged opposite to each other, and the first arc side and the second arc side are arranged opposite to each other. The tissue cutting side is used to cut tissue and/or cooperate with the clamp to clamp tissue, thereby achieving tissue separation and hemostasis. The first arc side and the second arc side are located on both sides of the tissue cutting side. The tissue cutting side has a curved surface structure. The first arc side and the second arc side are perpendicular to the plane formed by the two sides of the curved surface structure. The distal end of the blade body is offset from the central axis of the connecting rod. The back cutting side includes a first cutting surface and a second cutting surface. The first cutting surface is located between the first arc side and the second cutting surface to form a cutting edge with the second cutting surface. By designing the structure as such, the blade has a certain curvature, enabling it to be used in surgeries on human tissues with a certain curvature. This reduces the incision and thermal damage during surgery. At the same time, the cutting groove and other structures on the blade can be matched with the curvature parameters of the blade body, so that the large-curvature blade has stable vibration modes when operating at the designed resonant frequency, thus improving surgical safety.

**BRIEF DESCRIPTION OF THE DRAWINGS**

To more clearly illustrate the technical solutions of the embodiments of the present disclosure, the drawings used in the description of the embodiments or the prior art will be briefly introduced below. Obviously, the drawings described below are only some embodiments of the present disclosure. For those skilled in the art, other drawings can be obtained based on these drawings without creative effort.

FIG 1 is a structural diagram of the ultrasonic scalpel blade according to an embodiment of the present disclosure from a certain perspective.
FIG 2 is a structural diagram of the ultrasonic scalpel blade according to an embodiment of the present disclosure from another perspective.
FIG 3 is a structural diagram of a cross section of the blade body of the ultrasonic scalpel blade according to an embodiment of the present disclosure.
FIG 4 is a bottom view of the ultrasonic scalpel blade according to an embodiment of the present disclosure.
FIG 5 is a side view of the ultrasonic scalpel blade according to an embodiment of the present disclosure.
FIG 6 is a diagram of the structural parameters of the ultrasonic scalpel blade according to an embodiment of the present disclosure.
FIG 7 is a top view of the ultrasonic scalpel blade according to an embodiment of the present disclosure.
FIG 8 is another diagram of the structural parameters of the ultrasonic scalpel blade according to an embodiment of the present disclosure.
FIG 9 is a structural diagram of the ultrasonic scalpel according to an embodiment of the present disclosure.
FIG 10 is a structural diagram of another ultrasonic scalpel according to an embodiment of the present disclosure.
FIG 11 is a simulated diagram of the blade and blade shaft of the ultrasonic scalpel according to an embodiment of the present disclosure.
FIG 12 is a diagram of the vibration node of the blade and blade shaft of the ultrasonic scalpel according to an embodiment of the present disclosure.
FIG 13 is a diagram of the amplitude of the distal end of the ultrasonic scalpel blade according to an embodiment of the present disclosure.

List of reference numerals in the drawings:

100 - Blade
110 - Connecting Rod
120 - Blade Body
130 - Cutting Groove
121 - Tissue Cutting Side
122 - Back Cutting Side
123 - First Arc Side
124 - Second Arc Side
1221 - First Cutting Surface
1222 - Second Cutting Surface
1223 - Cutting Edge
1224 - First Transition Surface
1225 - Second Transition Surface
200 - Blade Shaft
300 - Clamp
400 - Transducer
500 - Sheath

**DETAILED DESCRIPTION OF THE EMBODIMENTS**

The technical solutions of the embodiments of the present application will be clearly described below with reference to the accompanying drawings. Obviously, the described embodiments are only some, not all, of the embodiments of the present application. Other embodiments obtained by those skilled in the art based on the embodiments of the present application without creative effort are all within the protection scope of the present application.

The terms such as "first", "second," and "third", etc., used in the description and claims of the present application are used to distinguish different objects, not to define a specific order. In the description of the present application, unless otherwise stated, "at least one" means one, two, or more.

In the embodiments of the present application, the terms such as "exemplary" or "for example", etc., are used to indicate that they are examples, illustrations, or descriptions. Any embodiment or design option that is described as "exemplary" or "for example" in the embodiments of the present application should not be construed as being more preferred or advantageous than other embodiments or design options. Specifically, the use of the terms such as "exemplary" or "for example", etc., is intended to present the relevant concepts in a specific manner.

The terminology used in the potion "DETAILED DESCRIPTION OF THE EMBODIMENTS" of the present application is only for explaining specific embodiments of the present application and is not intended to limit the present application. The embodiments of the present application will be described in detail below with reference to the accompanying drawings.

The ultrasonic scalpel is a surgical instrument that uses an ultrasonic transducer to generate ultrasonic vibration waves, which then drive the scalpel blade to cut and coagulate human tissue. It may be used in laparoscopic minimally invasive surgery. Driven by ultrasonic wave, the ultrasonic scalpel can quickly cut human tissue and coagulate incisions, reducing incision and tissue thermal damage during surgery, thereby improving surgical safety and postoperative recovery speed.

Because different parts of the human body have different tissue shapes, the cutting effect and thermal damage produced by the same type of ultrasonic scalpel blade on different tissue shapes will vary. Therefore, the same blade cannot achieve the same treatment effect on all tissue parts. In order to reduce the incision and thermal damage caused during the operation, different shapes of blades need to be used when cutting and coagulating tissues in different parts of the body.

Ultrasonic scalpels can be categorized into gun-type and scissor-type ultrasonic scalpels, etc., depending on the transducer and the connection mode between the blade and the transducer. Gun-type ultrasonic scalpels have a longer blade shaft between the blade and the transducer, while scissor-type ultrasonic scalpels have a shorter blade shaft between the blade and the transducer, or the blade is directly connected to the transducer. The present disclosure does not limit the type of ultrasonic scalpel used for the blade.

In the embodiment of the present disclosure, the ultrasonic scalpel is needed to operate on tissues with a large curvature, such as the thyroid gland. In this case, a straight blade or a blade with a small curvature causes significant incision and thermal damage to the tissue, which is detrimental to postoperative recovery. Therefore, a blade with a larger curvature is needed to reduce the incision and thermal damage caused by the surgery. However, since the ultrasonic scalpel is driven by ultrasonic vibration waves, different blade shapes, especially those with a large curvature, will affect the overall resonant frequency and vibration mode of the ultrasonic scalpel, thereby reducing the working stability of the ultrasonic scalpel and affecting its normal application.

It should be noted that the ultrasonic vibration waves emitted by the ultrasonic transducer are mechanical waves. Mechanical waves have the characteristic that their wave speed varies with the medium, and their wavelength is determined by both wave speed and frequency. Therefore, the wavelength of the mechanical wave is related to the material of the ultrasonic scalpel and the frequency of the mechanical wave. The ultrasonic transducer used in the ultrasonic scalpel is blade shaft-shaped. The blade of the ultrasonic scalpel can be connected to the ultrasonic transducer via the blade shaft, thereby driving the blade shaft and the blade of the ultrasonic scalpel vibrate at high frequency through the ultrasonic waves emitted by the ultrasonic transducer. In the embodiments of the present disclosure, the ultrasonic waves emitted by the ultrasonic transducer are longitudinal waves.

In some embodiments, the ultrasonic scalpel is designed to operate at a frequency of 55.6 kHz. The blade and the blade shaft of the ultrasonic scalpel are made of titanium alloy. The transmission speed of ultrasonic waves in the blade and the blade shaft is approximately 5000 m/s. Based on the relationship between mechanical wave frequency, wavelength, and speed, the wavelength of ultrasonic waves in the blade and blade shaft is approximately 90 mm.

It should be understood that the area where the ultrasonic scalpel ultimately achieves cutting and coagulation effects is the region from the farthest node to the antinode, corresponding to 1/4 wavelength. Taking the aforementioned wavelength of 90mm as an example, 1/4 wavelength is 22.5mm. To achieve sufficient physiological effects, a sufficiently large amplitude is required. It should be noted that the effective working length of an ultrasonic scalpel product is defined as the distance between the point of maximum amplitude at the farthest antinode and the point at 50% of the maximum amplitude. Therefore, based on the length of the region from the distal node to the antinode, the effective working length can be calculated to be 1/6 wavelength, approximately 15mm. However, an effective working length of 15mm is insufficient for all surgical scenarios, thus requiring a blade with a longer effective working length.

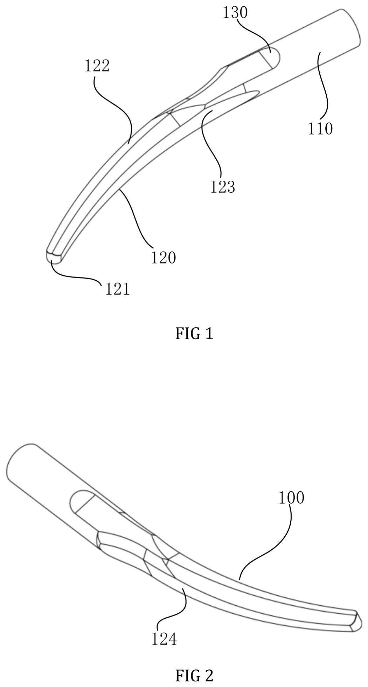

To address the aforementioned issues, the present disclosure proposes an ultrasonic scalpel blade, applicable to an ultrasonic scalpel including a clamp, to provide better treatment outcomes for human tissues with significant curvature, such as the thyroid gland. Referring to FIG 1 and FIG 2, which are structural diagrams of the ultrasonic scalpel blade from two different perspectives in an embodiment of the present application. As shown in FIG 1 and FIG 2, the ultrasonic scalpel blade 100 includes a connecting rod 110 and a blade body 120 sequentially from proximal to distal. The blade 100 has a cutting groove 130, which is disposed on the connecting rod 110 and extends towards the blade body 120. The cutting groove 130 can reduce unstable vibrations generated by the blade body 120 with large curvature.

It should be understood that the proximal and distal ends in the embodiments of the present disclosure are determined by the distance between the ultrasonic scalpel and the user during use. The end closer to the user is the proximal end, and the end farther from the user is the distal end. Furthermore, proximal and distal ends are relative concepts; that is, within the same component, the end closer to the user is the proximal end, and the end farther from the user is the distal end. The relationship between proximal and distal ends of different components needs to be determined based on the component arrangement. For example, taking the blade 100 and its connecting rod 110 and the blade body 120 in the embodiment of the present disclosure as an example, the ultrasonic scalpel blade 100 includes the connecting rod 110 and the blade body 120 sequentially from proximal to distal. The proximal position of the blade body 120 is located at the distal position of the connecting rod 110. When the ultrasonic scalpel has a blade shaft, the proximal position of the connecting rod 110 is located at the distal position of the blade shaft, while the proximal position of the blade shaft is located in the ultrasonic transducer, thereby connecting and fixing the blade shaft and the blade 100 to the ultrasonic transducer.

In some embodiments, the blade body 120 is the main component for realizing tissue cutting and coagulation functions. In order to realize the above functions, the blade body 120 includes a tissue cutting side 121, a back cutting side 122, a first arc side 123, and a second arc side 124, wherein the tissue cutting side 121 and the back cutting side 122 are arranged opposite to each other, and the first arc side 123 and the second arc side 124 are arranged opposite to each other, that is, the first arc side 123 and the second arc side 124 are located on both sides of the tissue cutting side 121 and the back cutting side 122, respectively.

For example, the tissue cutting side 121 may be a curved structure, and the first arc side 123 and the second arc side 124 are both perpendicular to the plane formed by the two sides of the curved structure. It should be understood that the tissue cutting side 121 can cut the diseased tissue under the drive of ultrasonic vibration waves, or it can be used in conjunction with the clamp to clamp the cut tissue to achieve tissue separation and hemostasis.

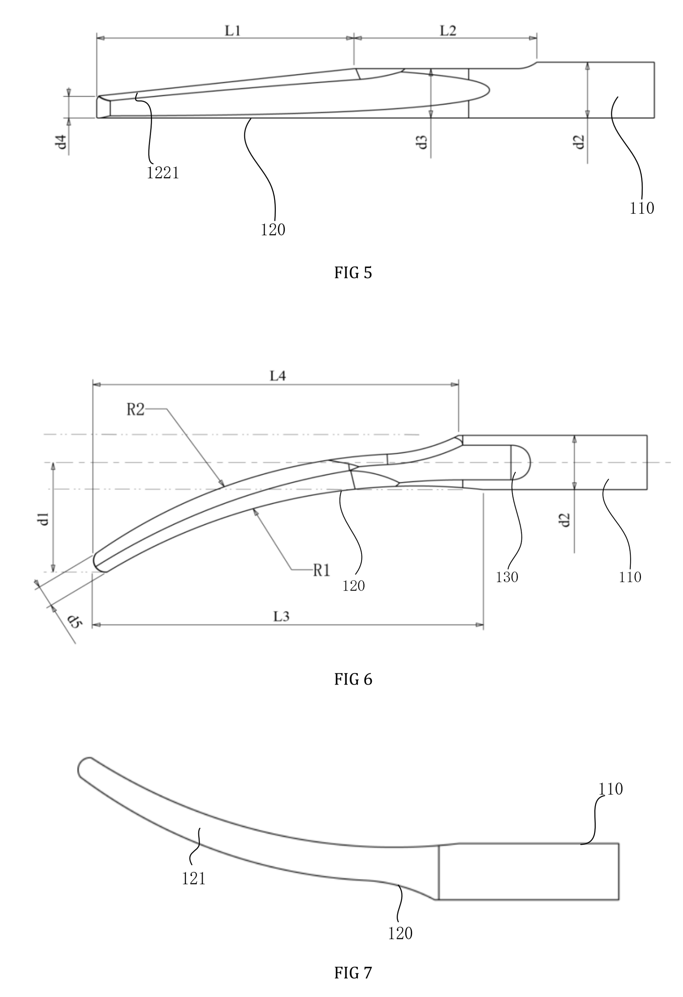

To enable surgery on tissues with large curvatures and reduce incision and thermal damage during surgery, the blade 100 needs to have a large curvature. Specifically, the distal end of the blade body 120 in the blade 100 deviates from the central axis of the connecting rod 110, that is, the blade body 120 is gradually away from the central axis of the connecting rod 110 from the proximal end to the distal end, giving the blade 100 a certain curvature. This results in fewer incision and thermal damage when cutting tissues with large curvatures, improving surgical outcomes. In some embodiments, the first vertical distance can be 3mm to 7mm. The first vertical distance is the vertical distance between the distal end of the blade body 120 and the central axis of the connecting rod 110. The degree of deviation between the blade body 120 and the connecting rod 110 can be specifically limited by the first vertical distance, thereby obtaining a blade 100 with a large curvature. In some embodiments, as shown in FIG 6, the first vertical distance can be d1.

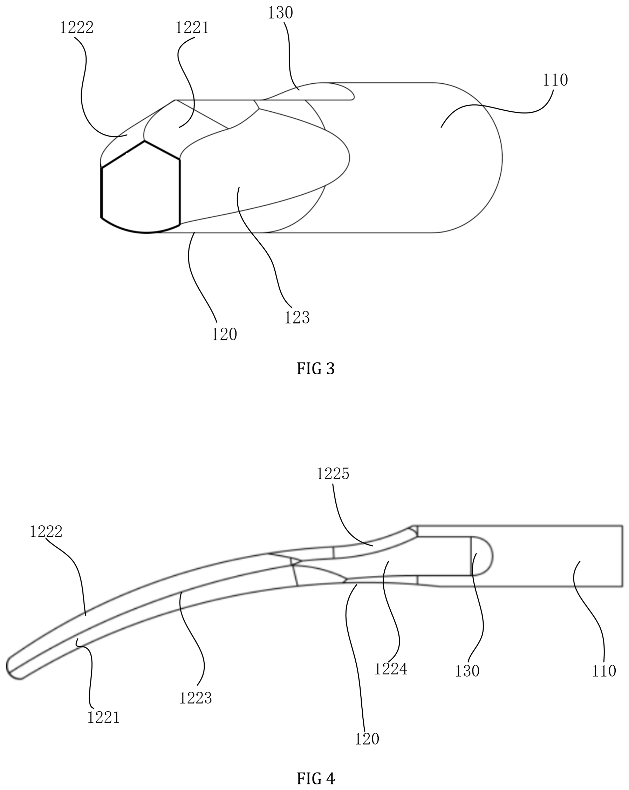

In some embodiments, the back cutting side 122 can also cut tissue, while simultaneously balancing the overall vibration stability of the ultrasonic scalpel blade 100. As shown in FIG 3 and FIG 4, the back cutting side 122 includes a first cutting surface 1221 and a second cutting surface 1222. The first cutting surface 1221 is located between the first arc side 123 and the second cutting surface 1222, and the second cutting surface 1222 is located between the first cutting surface 1221 and the second arc side 124. That is, the first cutting surface 1221 and the second cutting surface 1222 are arranged adjacent to each other, thereby forming the cutting edge 1223. It should be understood that, based on the fact that both the first arc side 123 and the second arc side 124 have a certain curvature, both the first cutting surface 1221 and the second cutting surface 1222 also have a certain curvature.

As shown in FIG 3, the cross sectional structure of the blade body 120 is similar to a "granary" type structure with a circular arc base, that is, a structure formed by a combination of a triangle and a rectangle. The angle formed by the two sides of the triangle that do not contact the rectangle is an obtuse angle, that is, the cutting edge angle of the cutting edge 1223 is an obtuse angle. For example, the cutting edge angle of the cutting edge 1223 can be 90°~150°, thereby providing better cutting ability for the cutting edge 1223 while reducing thermal damage to tissue caused by vibration during cutting.

Furthermore, the cutting surface length of the back cutting side 122 is 10mm to 14mm, and the transition surface length of the back cutting side 122 is 6mm to 10mm. The cutting surface length is the projected length of the cutting edge 1223 on the central axis of the connecting rod 110, and the transition surface length is the projected length of the first transition surface 1224 and the cutting groove 130 on the central axis of the connecting rod 110. By limiting the cutting edge angle of the cutting edge 1223 and the cutting surface length, the back cutting side 122 can meet the cutting effect required during surgery. The transition surface increases the overall length of the blade 100, and combined with the overall structural design of the blade 100, extends the effective working length of the blade 100.

Referring to FIG 5, the cutting surface length in this embodiment can be L1, and the transition surface length can be L2. For example, L1 and L2 can also be constrained by a first ratio, wherein the first ratio can be $\frac{L1}{L1+L2}$. In this embodiment, the first ratio satisfies $0.4 \leq \frac{L1}{L1+L2} \leq 0.8$, thereby further limiting the cutting surface length to match the overall structural design and extend the effective working length of the blade 100.

In some embodiments, in order to extend the effective working length of the blade 100, it is also necessary to configure the length structure of the first arc side 123 and the second arc side 124 so that the structure of the blade body 120 can extend the effective working length of the blade 100. For example, the first arc length of the first arc side 123 can be 25mm to 29mm, and on this basis, the second arc length of the second arc side 124 can be 23mm to 27mm, the length of the first arc side is 16mm to 20mm, and the length of the second arc side is 15mm to 19mm.

The length of the first arc side is the projected length of the first arc side 123 on the central axis of the connecting rod 110, and the length of the second arc side is the projected length of the second arc side 124 on the central axis of the connecting rod 110. By arranging the arc lengths of the two arc sides and their projected lengths on the central axis of the connecting rod 110, the length, curvature, and other structural features of the blade body 120 can be defined, thus ensuring that the blade 100 has a high degree of curvature while also possessing good working stability.

In some embodiments, the structure of the blade body 120 can be defined by specifying the relationship between the first arc length, the length of the first arc side, the second arc length, and the length of the second arc side. As shown in FIG 6, the first arc length is R1, the length of the first arc side is L3, the second arc length is R2, and the length of the second arc side is L4. For example, the shape of the blade body 120 can be defined by the difference between the ratio of R1 and L3 and the ratio of R2 and L4, i.e., the value of $\frac{R1}{L3} - \frac{R2}{L4}$.

R1 represents the first arc length, R2 represents the second arc length, L3 represents the length of the first arc side, and L4 represents the length of the second arc side. In some embodiments, since the blade body 120 is gradually thinner, the curvature of the second arc side 124 is different from that of the first arc side 123, thereby making the distance between the first arc side 123 and the second arc side 124 smaller in the direction from the proximal end to the distal end. For example, the length and curvature shape of the first arc side 123 and the second arc side 124 can be defined by the formula $\frac{R1}{L3} - \frac{R2}{L4} \leq 0.1$. Therefore, by limiting the ratio of the arc length and projected length between the first arc side 123 and the second arc side 124, the curvature and shape of the blade body 120 can be defined, thereby improving the vibration stability of the blade 100 through the overall shape arrangement of the blade 100.

It should be noted that, since the effective working length and curvature of the blade 100 are both related to the wavelength and frequency of the ultrasonic waves driving the blade 100, the above structure is designed to reduce the vibration frequency of the ultrasonic waves transmitted through the blade shaft to the blade 100, thereby increasing the ultrasonic wavelength at the distal end of the blade 100 and increasing the effective working length of the blade 100. Through this configuration, the farthest quarter wavelength length of the ultrasonic scalpel can be increased to 30 mm, thereby increasing the effective working length of the blade 100 to 20 mm.

By offsetting the distal end of the blade body 120 from the central axis of the connecting rod 110, the blade body 120 has a certain curvature, thereby enabling the blade 100 to be used in surgeries on human tissues with a certain curvature, reducing incision and thermal damage during operation. The cutting groove 130 on the blade 100 can be matched with the curvature parameters of the blade body 120, ensuring stable vibration modes of the large-curvature blade 100 when operating at its designed resonant frequency, thus improving surgical safety.

Because the back cutting side 122 is provided with a cutting edge 1223, it will affect the smoothness of the transition area at the connection position between the back cutting side 122 and the connecting rod 110, thereby affecting the overall vibration stability of the blade 100. In some embodiments, as shown in FIG 4, the back cutting side 122 also includes a first transition surface 1224 and a second transition surface 1225, wherein the first transition surface 1224 is disposed between the first cutting surface 1221, the second transition surface 1225 and the cutting groove 130. Therefore, the first transition surface 1224 is a plane formed by the adjacent edges of the first cutting surface 1221, the second transition surface 1225 and the cutting groove 130, so as to provide a transition surface from the first cutting surface 1221 to the cutting groove 130.

The second transition surface 1225 is disposed between the second cutting surface 1222, the second arc side 124 and the first transition surface 1224. It should be understood that the second transition surface 1225 is an arc surface structure and is formed by the adjacent edges of the second cutting surface 1222, the second arc side 124 and the first transition surface 1224. The curvature of the second transition surface 1225 is the same as the curvature of the second arc side 124 that contacts the second transition surface 1225, and therefore the second transition surface 1225 is a transition surface from the second cutting surface 1222 to the connecting rod 110.

For example, the length of the blade body can be 15mm to 19mm, and the length of the connecting rod 110 can be 1mm to 5mm, wherein the length of the blade body is the projected length of the arc side of the blade body 120 onto the central axis of the connecting rod 110. It should be understood that the connecting rod 110 is the straight section of the blade 100, that is, a structure coaxially arranged with the blade in the blade 100, and the connecting rod 110 mainly serves as a transition from the blade shaft to the blade 100.

The blade body 120 of the above structure enables the blade 100 to have a length greater than or equal to 15mm in the direction of the central axis of the connecting rod 110. Furthermore, the effective working length of the blade 100 is increased by the arc-shaped blade body 120. Moreover, through the aforementioned structural arrangement, the blade 100 can improve its working stability while extending its effective working length.

By setting two transition surfaces on the back cutting side 122, the overall length of the blade body 120 is increased, making the effective working distance of the blade body 120 closer to the distal end. At the same time, the setting of the transition surfaces can optimize the shape of the first cutting surface 1221, the second cutting surface 1222 and the cutting edge 1223, so that the blade body 120 smoothly transitions with the connecting rod 110 and the cutting groove 130, thereby reducing the unevenness of the blade 100 and improving the vibration stability of the blade 100 during operation.

In some embodiments, as shown in FIG 7, in order to provide better tissue cutting effect, the width of the blade body 120 gradually decreases from the proximal end to the distal end, wherein the width of the blade body 120 is the vertical distance between the first arc side 123 and the second arc side 124, and the width of the blade body 120 has a maximum value at the proximal end of the blade body 120 and a minimum value at the distal end of the blade body 120.

It should be understood that although the width of the blade body 120 gradually decreases from the proximal end to the distal end, that is, the first arc side 123 and the second arc side 124 gradually approach each other from the proximal end to the distal end, in the cross section of the blade body 120, the edge line corresponding to the first arc side 123 is parallel to the edge line corresponding to the second arc side 124, thereby maintaining the shape of the blade 100.

For example, the width of the blade body 120 can be 0.5mm to 3mm. It should be noted that the above range is the common width range of the proximal end and distal end of the blade body 120. Because the width of the blade body 120 gradually decreases from the proximal end to the distal end, the minimum width of the proximal end of the blade body 120 is greater than or equal to the maximum width of the distal end of the blade body 120. The gradually thinning blade body 120 allows the distal end of the blade 100 to have a better cutting effect. At the same time, during the cutting process, the smaller blade width can also reduce the surgical incision, facilitating postoperative recovery for the patient.

In some embodiments, the diameter of the first cross section of the connecting rod 110 is 2mm to 3mm, and the width of the second end face of the distal end of the blade body 120 is 0.5mm to 2mm. The first cross section diameter is the diameter of the cross section of the connecting rod 110, and the second end face width is the width of the distal end of the blade body 120. In some embodiments, as shown in FIG 6, the width of the second end face can be d5. Because the proximal end of the connecting rod 110 is adjacent to the proximal end of the blade body 120, for a smooth transition between the blade body 120 and the connecting rod 110, the width of the proximal end of the blade body 120 can be the same as the diameter of the first cross section, i.e., the width of the proximal end of the blade body 120 is 2mm to 3mm. It should be understood that when the width of the proximal end of the blade body 120 is 2mm, the width of the second end face cannot be 2mm, and only when the width of the proximal end of the blade body 120 is greater than 2mm can the width of the second end face be 2mm.

Because the cutting groove 130 in the blade 100 extends from the connecting rod 110 to the blade body 120, and the cutting groove 130 has a certain depth, the height at the proximal end of the blade body 120 is less than the width at the proximal end of the blade body 120. For example, the height of the first end face of the proximal end of the blade body is 1.8mm to 3mm, and the depth of the cutting groove 130 can be 0.2mm to 0.4mm. The cutting groove 130 can balance the unstable vibration generated by the cutting edge 1223 on the back cutting side 122. It should be understood that the height of the first end face is the vertical distance between the tissue cutting side 121 and the back cutting side 122 at the proximal position of the blade body 120. In some embodiments, the height of the first end face can be the vertical distance between the bottom of the curved structure of the tissue cutting side 121 and the first transition surface 1224.

In some embodiments, the width of the blade body 120 gradually decreases from proximal to distal, and the height thereof also gradually decreases. Specifically, the height of the second end face at the distal end of the blade body 120 can be 0.5mm to 2mm. The height of the second end face is the vertical distance between the tissue cutting side 121 and the back cutting side 122 at the distal position of the blade body. Since the back cutting side 122 at the distal end of the blade body 120 includes the cutting edge 1223, the height of the second end face can also be the vertical distance between the cutting edge 1223 and the bottom of the curved structure of the tissue cutting side 121. This further limits the overall structural size of the blade 100. By using a blade body 120 with a smaller width and height at its distal end, the incision caused by the blade 100 during surgery is smaller, making the blade 100 more suitable for minimally invasive surgery.

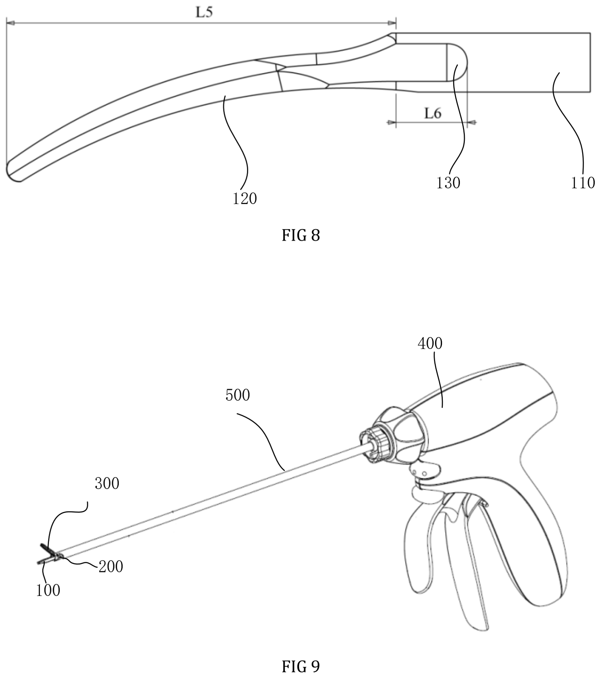

As shown in FIG 5 and FIG 8, in some embodiments, the length of the blade body can be L5, the length of the connecting rod can be L6, the diameter of the first cross section can be d2, the height of the first end face can be d3, and the height of the second end face can be d4. To ensure the overall structure of the blade 100 maintains vibration stability during operation, the dimensions L5, L6, d2, d3, and d4 are all related. For example, they may satisfy $\frac{L5}{L5+L6} - \frac{d3-d4}{d2-d4} \leq 0.1$. By utilizing the dimensional relationships between different structural positions of the blade 100, the overall structure of the blade 100 can meet the operating frequency of the ultrasonic scalpel and maintain operational stability.

Based on any of the aforementioned ultrasonic scalpel blades, as shown in FIG 9, the present disclosure also provides an ultrasonic scalpel, including a clamp 300, any one of the ultrasonic scalpel blades 100 as described above, a blade shaft 200, a transducer 400, and a sheath 500. The blade 100 includes a connecting rod 110 and a blade body 120. The connecting rod 110 is located at the distal end of the blade shaft 200, and the blade body 120 is located at the distal end of the connecting rod 110. The proximal end of the blade shaft 200 is connected to the transducer 400. The sheath 500 is sleeved outside the blade shaft 200. The proximal end of the sheath 500 is connected to the transducer 400, and the distal end of the sheath 500 is connected to the clamp 300.

The blade 100 and the blade shaft 200 can be made of the same material and integrally formed. When replacing the blade 100, the blade shaft 200 shall be replaced along with the blade 100. Since the clamp 300 can cooperate with the blade 100 to perform clamping, the clamp 300 is hinged to the distal end of the sheath 500, allowing the clamp 300 to rotate to cooperate with the blade 100 in clamping tissues and performing other operations.

Since the blade 100 and the blade shaft 200 can be made of the same material and integrally formed, the structure of the blade 100 in the aforementioned embodiment provides good machinability under condition that the blade has large curvature, making it easy to process and obtain, and reducing the processing difficulty of the ultrasonic scalpel.

It should be understood that the vibration stability of the ultrasonic scalpel can be evaluated by the ratio of the radial to axial vibration of the blade shaft 200. The smaller this ratio, the larger the axial component of the vibration (i.e., the component of vibration in the direction of the blade shaft 200), and the higher the vibration stability. Conversely, the larger the radial component of the vibration, the greater the bending or torsional vibration of the shaft 200, and the worse the stability. However, the blade 100 requires a certain radial vibration component to achieve the cutting and coagulation effect, and therefore, the blade 100 needs to be configured as described above to achieve this effect.

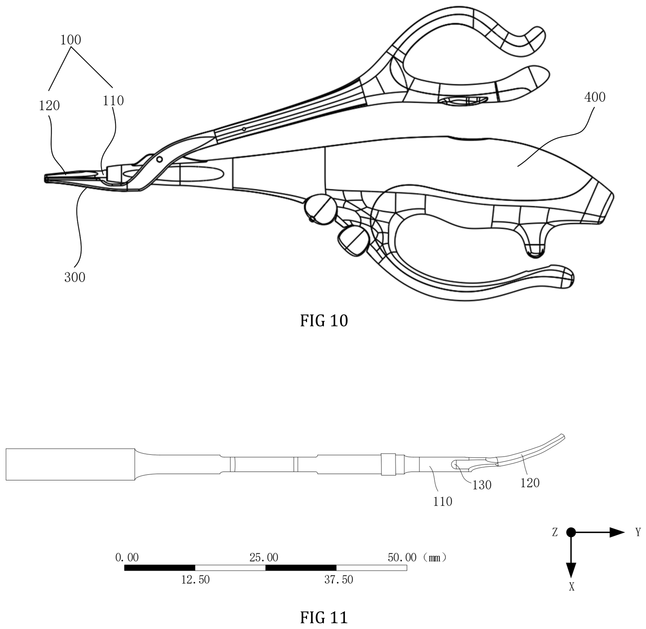

Based on any of the aforementioned ultrasonic scalpel blades, as shown in FIG 10, the present disclosure also provides an ultrasonic scalpel, which is a scissor-type ultrasonic scalpel. Specifically, the ultrasonic scalpel includes a clamp 300, and also includes any of the aforementioned ultrasonic scalpel blades 100 and a transducer 400. The blade 100 includes a connecting rod 110 and a blade body 120, with the blade body 120 being disposed at the distal end of the connecting rod 110. The proximal end of the connecting rod 110 is connected to the transducer 400, and the clamp 300 is hinged to the transducer 400 so that the clamp 300 and the blade body 120 cooperate to clamp tissue.

The blade body 120 is directly connected to the transducer 400 via the connecting rod 110, while the clamp 300 is hinged to the housing of the transducer 400, allowing the clamp 300 to rotate at a certain angle, thereby cooperating with the blade body 120 to clamp and release the clamped tissue.

In some embodiments, as shown in FIG 11 to FIG 13, taking a gun-type ultrasonic scalpel as an example, its structural features can be verified by modeling and simulating the ultrasonic scalpel. For example, as shown in FIG 11, using the aforementioned embodiment of the blade 100, a blade 100 can be constructed with a first arc length of 27mm, a second arc length of 25mm, a first arc side length of 18mm, a second arc side length of 17mm, a cutting surface length of 12mm, a transition surface length of 8mm, a blade body length of 17mm, a connecting rod length of 3mm, a first vertical distance of 5.5mm, a first cross sectional diameter of 2.6mm, a first end face height of 2.3mm, a second end face height of 1mm, and a second end face width of 1mm. Combined with the blade shaft 200, this forms an ultrasonic scalpel with a total length of 100mm, consisting of the blade shaft 200 and the blade 100.

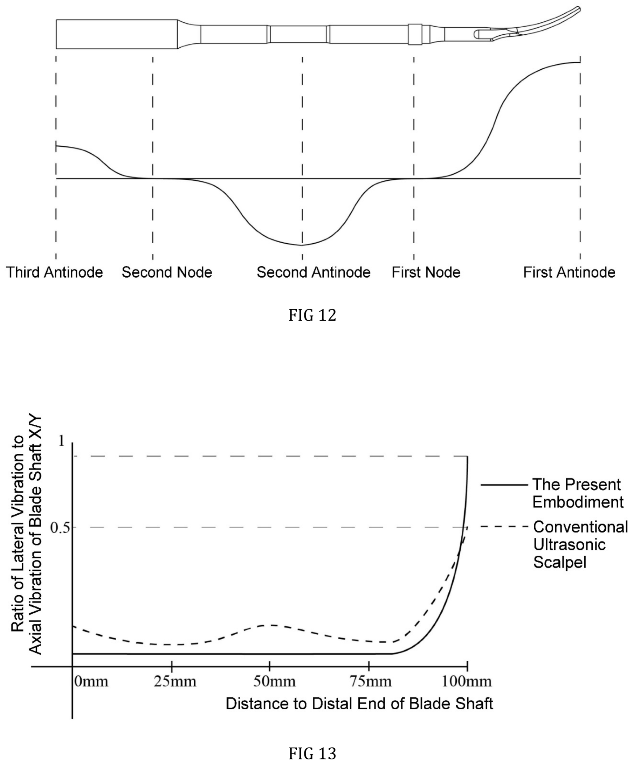

As shown in FIG 12, to improve the vibration stability of the ultrasonic scalpel, the position where the proximal end of the blade shaft 200 connects to the transducer 400 is the antinode, i.e., the third antinode, while the farthest position of the blade 100 is also the antinode, i.e., the first antinode, which is the position with the largest amplitude. Between the two antinodes, from the distal end to the proximal end, are the first node, the second antinode, and the second node, respectively. It should be understood that the third antinode is the antinode with the smallest amplitude, and the vibration of the blade shaft 200 and the blade 100 is amplified step by step from the proximal end to the distal end.

In this embodiment, the plane perpendicular to the central axis at the proximal antinode is used as the proximal connection surface, and this is used as the reference surface to simulate the blade shaft 200 and the blade 100. For ease of description, as shown in FIG 11, a three-dimensional coordinate system is established. The Y-axis direction is the axial direction of the blade shaft 200; the X-axis direction is perpendicular to the blade shaft 200, i.e., the radial direction of the blade shaft 200, and the X-axis is in the same direction as the bending direction of the scalpel blade 100; the Z-axis direction is perpendicular to the plane formed by the X-axis and Y-axis. Vibration morphology analysis of the blade shaft 200 and the blade 100 yields the average vibration amplitude along the Y-axis and X-axis directions on the proximal end face of the blade shaft 200. As shown in FIG 13, in this embodiment, the amplitude ratio of the ultrasonic scalpel at the distal end of the blade 200 is approximately X/Y = 1/1.2, and the amplitude ratio at the antinode of the proximal end face of the blade shaft 200 is approximately X/Y = 1/200.

In this embodiment, during the overall vibration morphology analysis of the blade 100 and blade shaft 200, the blade body vibrates only along the axial direction at any position, and its radial amplitude does not change with the ultrasonic waveform. Even in the antinode region where the vibration amplitude is relatively largest, the radial vibration ratio is still less than 1%, which can be ignored. Traditional ultrasonic scalpels, however, exhibit fluctuations in radial amplitude along the blade shaft with the ultrasonic waveform. Furthermore, areas with larger vibration amplitudes on the blade shaft have higher radial vibration ratios and poorer stability. Therefore, the design structure in this embodiment improves the vibration stability of the blade shaft 200 and increases the radial amplitude on the blade 100. This allows the ultrasonic scalpel illustrated in this embodiment to achieve good therapeutic effects while reducing the impact of the higher radial amplitude of the blade 100 on the vibration stability of the blade shaft 200, thus improving the overall operational stability of the ultrasonic scalpel.

As can be seen from the above technical solutions, the present disclosure provides an ultrasonic scalpel blade, applied to an ultrasonic scalpel including a clamp 300. The blade 100 includes a connecting rod 110 and a blade body 120 sequentially from the proximal end to the distal end. The blade 100 is provided with a cutting groove 130, which is located on the connecting rod 110 and extends towards the blade body 120. The blade body 120 includes a tissue cutting side 121, a back cutting side 122, a first arc side 123, and a second arc side 124. The tissue cutting side 121 and the back cutting side 122 are arranged opposite to each other, and the first arc side 123 and the second arc side 124 are arranged opposite to each other. The tissue cutting side 121 is used to cut tissue and/or cooperate with the clamp 300 to clamp tissue, thereby achieving tissue separation and hemostasis. The first arc side 123 and the second arc side 124 are located on both sides of the tissue cutting side 121. The tissue cutting side 121 has a curved surface structure. The first arc side 123 and the second arc side 124 are perpendicular to the plane formed by the two sides of the curved surface structure. The distal end of the blade body 120 is offset from the central axis of the connecting rod 110. The back cutting side 122 includes a first cutting surface 1221 and a second cutting surface 1222. The first cutting surface 1221 is located between the first arc side 123 and the second cutting surface 1222 to form a cutting edge 1223 with the second cutting surface 1222. By designing the structure as such, the blade 100 has a certain curvature, enabling it to be used in surgeries on human tissues with a certain curvature. This reduces the incision and thermal damage during surgery. At the same time, the cutting groove 130 and other structures on the blade 100 can be matched with the curvature parameters of the blade body 120, so that the large-curvature blade 100 has stable vibration modes when operating at the designed resonant frequency, thus improving surgical safety.

Similar parts between the embodiments provided in the present application can be referred to across different embodiments. The specific embodiments provided above are only a few examples under the overall concept of the present application and do not constitute a limitation on the scope of the present application. For those skilled in the art, any other embodiments derived from the solution of the present application without creative effort shall fall within the scope of the present application.

## Claims

1. An ultrasonic scalpel blade applied to an ultrasonic scalpel comprising a clamp, the blade comprising a connecting rod and a blade body sequentially from a proximal end to a distal end,

   wherein the blade is provided with a cutting groove, which is located on the connecting rod and extends towards the blade body;
   wherein the blade body includes a tissue cutting side, a back cutting side, a first arc side, and a second arc side, the tissue cutting side and the back cutting side are arranged opposite to each other, and the first arc side and the second arc side are arranged opposite to each other, wherein the tissue cutting side is configured to cut tissue and/or cooperate with the clamp to clamp tissue in order to achieve tissue separation and hemostasis;
   wherein the first arc side and the second arc side are located on two sides of the tissue cutting side, the tissue cutting side has a curved surface structure, the first arc side and the second arc side are perpendicular to the plane formed by the two sides of the curved surface structure, and the distal end of the blade body is offset from a central axis of the connecting rod; and
   wherein the back cutting side includes a first cutting surface and a second cutting surface, with the first cutting surface being located between the first arc side and the second cutting surface, and the second cutting surface being located between the first cutting surface and the second arc side, and wherein the first cutting surface and the second cutting surface form a cutting edge.

2. The ultrasonic scalpel blade according to claim 1, wherein the back cutting side further comprises a first transition surface and a second transition surface;

   the first transition surface is disposed between the first cutting surface, the second transition surface and the cutting groove, and the first transition surface is a plane formed by the first cutting surface, the second transition surface and the cutting groove; and
   the second transition surface is disposed between the second cutting surface, the second arc side and the first transition surface, and the second transition surface is an arc surface formed by the second cutting surface, the second arc side and the first transition surface, wherein the curvature of the second transition surface is the same as the curvature of the second arc side.

**3.** The ultrasonic scalpel blade according to claim 2, wherein the cutting edge angle is in the range of 90° to 150°, the cutting surface length of the back cutting side is in the range of 10mm to 14mm, and the transition surface length of the back cutting side is in the range of 6mm to 10mm, wherein the cutting surface length is a projected length of the cutting edge on the central axis of the connecting rod, and the transition surface length is a projected length of the first transition surface and the cutting groove on the central axis of the connecting rod.

**4.** The ultrasonic scalpel blade according to claim 3, satisfying the formula:

$$0.4 \leq \frac{L1}{L1+L2} \leq 0.8;$$

wherein, L1 represents the cutting surface length, and L2 represents the transition surface length.

**5.** The ultrasonic scalpel blade according to claim 1, wherein a width of the blade body is a vertical distance between the first arc side and the second arc side, and the width of the blade body is in the range of 0.5mm to 3mm; the width of the blade body has a maximum value at the proximal end of the blade body and a minimum value at the distal end of the blade body, and the width of the blade body gradually decreases from the proximal end to the distal end of the blade body;
a first arc length of the first arc side is in the range of 25mm to 29mm, and a second arc length of the second arc side is in the range of 23mm to 27mm; a first vertical distance is in the range of 3mm to 7mm; a length of the first arc side is in the range of 16mm to 20mm, and a length of the second arc side is in the range of 15mm to 19mm; wherein the first vertical distance is a vertical distance between the distal end of the blade body and the central axis of the connecting rod, the length of the first arc side is a projected length of the first arc side on the central axis of the connecting rod, and the length of the second arc side is a projected length of the second arc side on the central axis of the connecting rod.

**6.** The ultrasonic scalpel blade according to claim 5, satisfying the formula:

$$\frac{R1}{L3} - \frac{R2}{L4} \leq 0.1;$$

wherein, R1 represents the first arc length, R2 represents the second arc length, L3 represents the length of the first arc side, and L4 represents the length of the second arc side.

**7.** The ultrasonic scalpel blade according to claim 5, wherein a length of the blade body is in the range of 15mm to 19mm, and a length of the connecting rod is in the range of 1mm to 5mm, wherein the length of the blade body is a projection length of the arc side of the blade body on the central axis of the connecting rod.

**8.** The ultrasonic scalpel blade according to claim 7, wherein a diameter of a first cross section of the connecting rod is in the range of 2mm to 3mm, a height of a first end face of the proximal end of the blade body is in the range of 1.8mm to 3mm, a height of a second end face of the distal end of the blade body is in the range of 0.5mm to 2mm, and a width of the second end face of the distal end of the blade body is in the range of 0.5mm to 2mm;
wherein the diameter of the first cross section is the diameter of the cross section of the connecting rod, the height of the first end face is a vertical distance between the tissue cutting side and the back cutting side at the proximal position of the blade body, the height of the second end face is a vertical distance between the tissue cutting side and the back cutting side at the distal position of the blade body, and the width of the second end face is the width of the distal end of the blade body.

**9.** The ultrasonic scalpel blade according to claim 8, satisfying the formula:

$$\frac{L5}{L5+L6} - \frac{d3-d4}{d2-d4} \leq 0.1;$$

wherein L5 represents the length of the blade body, L6 represents the length of the connecting rod, d2 represents the diameter of the first cross section, d3 represents the height of the first end face, and d4 represents the height of the second end face.

**10.** An ultrasonic scalpel, comprising a clamp, the ultrasonic scalpel blade according to any one of claims 1-9, a blade shaft, a transducer, and a sheath,

wherein the blade includes a connecting rod and a blade body, the connecting rod is disposed at the distal end of the blade shaft, and the blade body is disposed at the distal end of the connecting rod; and
wherein the proximal end of the blade shaft is connected to the transducer, the sheath is sleeved outside the blade shaft, the proximal end of the sheath is connected to the transducer, and the distal end of the sheath is connected to the clamp.

**11.** An ultrasonic scalpel, comprising a clamp, the ultrasonic scalpel blade according to any one of claims

**EP 4 721 681 A1**

1-9, and a transducer,

wherein the blade includes a connecting rod and a blade body, with the blade body being disposed at the distal end of the connecting rod; and

wherein the proximal end of the connecting rod is connected to the transducer, and the clamp is hinged to the transducer so that the clamp cooperates with the blade body to clamp tissue.

FIG 1

FIG 2

1222    1221    130                    110

120    123

FIG 3

1225

1222

1221    1223    120    1224    130    110

FIG 4

FIG 5

FIG 6

FIG 7

FIG 8

FIG 9

FIG 10

0.00        25.00        50.00 (mm)

12.50        37.50

FIG 11

Third Antinode    Second Node    Second Antinode    First Node    First Antinode

FIG 12

FIG 13

## INTERNATIONAL SEARCH REPORT

| | |
|---|---|
| International application No. | |
| | **PCT/CN2024/101863** |

| A. | CLASSIFICATION OF SUBJECT MATTER |
|---|---|

A61B 17/32(2006.01)i

According to International Patent Classification (IPC) or to both national classification and IPC

| B. | FIELDS SEARCHED |
|---|---|

Minimum documentation searched (classification system followed by classification symbols)

IPC: A61B

Documentation searched other than minimum documentation to the extent that such documents are included in the fields searched

Electronic data base consulted during the international search (name of data base and, where practicable, search terms used)

CNABS, CNTXT, DWPI, WPABSC, ENTXT: 以诺康医疗科技, 超声, 手术刀, 弯曲, 弧度, 弧形, 切槽, 剪切, 组织, 分离, 止血, 切割, 过渡, 稳定, 振动, ultrasonic, blade, curved, edge, cutter, waveguide, bend+, blood

| C. | DOCUMENTS CONSIDERED TO BE RELEVANT |
|---|---|

| Category* | Citation of document, with indication, where appropriate, of the relevant passages | Relevant to claim No. |
|---|---|---|
| PX | CN 116983053 A (YINUOKANG MEDICAL TECHNOLOGY (SUZHOU) CO., LTD.) 03 November 2023 (2023-11-03) claims 1-11 | 1-11 |
| X | CN 107750141 A (ETHICON LLC) 02 March 2018 (2018-03-02) description, paragraphs 41-96, and figures 1-31 | 1-4, 10, 11 |
| A | CN 107847388 A (REACH SURGICAL, INC.) 27 March 2018 (2018-03-27) entire document | 1-11 |
| A | CN 113017777 A (INNOLCON MEDICAL TECHNOLOGY (SUZHOU) CO., LTD.) 25 June 2021 (2021-06-25) entire document | 1-11 |
| A | CN 116392209 A (WUHAN MINDRAY MEDICAL TECHNOLOGY RESEARCH INSTITUTE CO., LTD. et al.) 07 July 2023 (2023-07-07) entire document | 1-11 |
| A | US 2019133633 A1 (ETHICON L.L.C.) 09 May 2019 (2019-05-09) entire document | 1-11 |

☐ Further documents are listed in the continuation of Box C.    ☑ See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "D" document cited by the applicant in the international application | "X" document of particular relevance; the claimed invention cannot be considered novel or cannot be considered to involve an inventive step when the document is taken alone |
| "E" earlier application or patent but published on or after the international filing date | |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **05 September 2024** | **12 September 2024** |

| Name and mailing address of the ISA/CN | Authorized officer |
|---|---|
| **China National Intellectual Property Administration (ISA/CN)** **China No. 6, Xitucheng Road, Jimenqiao, Haidian District, Beijing 100088** | |
| | Telephone No. |

Form PCT/ISA/210 (second sheet) (July 2022)

## INTERNATIONAL SEARCH REPORT
### Information on patent family members

| International application No. |
| --- |
| **PCT/CN2024/101863** |

| Patent document cited in search report | | | Publication date (day/month/year) | Patent family member(s) | | | Publication date (day/month/year) |
| --- | --- | --- | --- | --- | --- | --- | --- |
| CN | 116983053 | A | 03 November 2023 | None | | | |
| CN | 107750141 | A | 02 March 2018 | US | 2016367281 | A1 | 22 December 2016 |
| | | | | US | 11020140 | B2 | 01 June 2021 |
| | | | | US | 2021315605 | A1 | 14 October 2021 |
| | | | | JP | 2018518286 | A | 12 July 2018 |
| | | | | JP | 7019424 | B2 | 15 February 2022 |
| | | | | WO | 2016204999 | A1 | 22 December 2016 |
| | | | | EP | 3310274 | A1 | 25 April 2018 |
| CN | 107847388 | A | 27 March 2018 | EP | 3334399 | A1 | 20 June 2018 |
| | | | | EP | 3334399 | B1 | 17 June 2020 |
| | | | | EP | 3718494 | A1 | 07 October 2020 |
| | | | | EP | 3718494 | B1 | 16 November 2022 |
| | | | | WO | 2017027853 | A1 | 16 February 2017 |
| | | | | US | 2018242997 | A1 | 30 August 2018 |
| | | | | US | 11058449 | B2 | 13 July 2021 |
| | | | | US | 2022039825 | A1 | 10 February 2022 |
| CN | 113017777 | A | 25 June 2021 | None | | | |
| CN | 116392209 | A | 07 July 2023 | None | | | |
| US | 2019133633 | A1 | 09 May 2019 | US | 2015119915 | A1 | 30 April 2015 |
| | | | | US | 9883884 | B2 | 06 February 2018 |
| | | | | US | 10828057 | B2 | 10 November 2020 |
| | | | | US | 2008234710 | A1 | 25 September 2008 |
| | | | | US | 8911460 | B2 | 16 December 2014 |
| | | | | US | 2015119914 | A1 | 30 April 2015 |
| | | | | US | 9987033 | B2 | 05 June 2018 |

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 202311243079